# EUROPEAN PATENT APPLICATION

(11) **EP 4 806 355 A1**
(43) Date of publication of application: **16.09.2026**
(21) Application number: 25163319.4
(22) Date of filing: 12.03.2025
(51) Int. Cl.: A61B 5/259, A61B 5/00

(54) **REPOSITIONABLE MEDICAL DEVICE**

(71) Applicant: Koninklijke Philips N.V., 5656 AE Eindhoven (NL)
(72) Inventor: GELISSEN, Jozef Hubertus, 5656 AG Eindhoven (NL)
(74) Representative: Philips Intellectual Property & Standards

(57) **Abstract**

The present invention relates to repositionable medical device for application to a user's skin comprising a carrier layer having an application surface configured to be directed towards the skin when applied to the user, a functional unit embedded within the carrier layer or positioned on the application surface, and a release layer having a first release surface and a second release surface. The second release surface is configured to adhere to the application surface of the carrier layer. The first release surface is configured to adhere to i) the skin when applied to the user, wherein detachment of the release layer exposes an adhesive portion of the application surface, or ii) an additional release layer, wherein detachment of the additional release layer exposes the first release surface allowing a renewed application of the medical device to the user's skin.

## Description

### FIELD OF THE INVENTION

The present invention relates to a repositionable medical device for application to a user's skin.

### BACKGROUND OF THE INVENTION

In recent years, the medical field has seen a significant increase in the development and use of wearable medical devices for a variety of health applications. These devices range from conventional dry electrodes to transdermal drug delivery systems and biosensing patches that track vital signs such as heart rate, oxygen levels and hydration status. The convenience and effectiveness of such devices have made them increasingly popular with healthcare providers and patients alike.

Various approaches to improve attachment of these medical devices to the skin have been explored. WO 2014/084401 A1, for example, discloses a medical patch having applications such as protection of the skin and administration of a drug into the living body through the skin surface.

While high adhesive strength is important for long-term wearability, there are several drawbacks and limitations. In particular, many currently available medical patches and adhesive devices are not designed to be removed once applied and be re-applied again, limiting the ability to reposition. This is a significant challenge, as incorrect placement can result in suboptimal sensor readings, discomfort, or even skin irritation. In addition, certain applications, such as continuous glucose monitoring, require precise positioning to function effectively, making the inability to reposition the device a significant limitation.

Many adhesives lose their effectiveness after a single use, leading to reduced adhesion and potential device failure. Factors such as sweat, moisture, and body movement can compromise adhesion over time, further exacerbating the problem. In particular, patients with sensitive skin may experience redness, rashes, or allergic reactions due to the materials used in conventional adhesive patches.

Overall, while wearable medical devices provide advanced patient monitoring and treatment, the inability to reposition these devices without compromising their functionality remains a significant issue.

### SUMMARY OF THE INVENTION

It is an object of the present invention to improve the repositionability of medical adhesive patches without compromising their adhesion, functionality or user comfort.

In a first aspect of the present invention, a repositionable medical device for application to a user's skin is presented, the device comprising:
- a carrier layer having an application surface configured to be directed towards the skin when applied to the user,
- a functional unit embedded within the carrier layer or positioned on the application surface, and
- a release layer having a first release surface and a second release surface;
   wherein the second release surface is configured to adhere to the application surface of the carrier layer, and
   wherein the first release surface is configured to adhere to:
      i) the skin when applied to the user, wherein detachment of the release layer exposes an adhesive portion of the application surface, or
      ii) an additional release layer, wherein detachment of the additional release layer exposes the first release surface allowing a renewed application of the medical device to the user's skin.

The present invention is based on the idea that a medical device can include a multi-layer adhesive system that allows for repositioning while maintaining good adhesion and minimizing skin irritation. This is particularly relevant for wearable medical devices such as biosensors, drug delivery patches, and dry electrodes that require precise placement for optimal functionality. A key challenge with wearable devices is that while good adhesives are necessary for long-term attachment, they tend to accumulate dead skin cells, hair, and other biomaterials, leading to reduced adhesion over time and limiting the ability to reposition. To address this, the present invention introduces a layered (or multi-layer) adhesive system in which an additional layer of skin adhesive can be removed in a tear-off fashion, ensuring that the device can be repositioned without compromising adhesion or requiring a new device.

The present invention thus addresses the limitations of existing medical patches by enabling extended wear and repositionability without sacrificing adhesion performance. The proposed repositionable medical device maintains secure adhesion while allowing for multiple applications, which improves user experience and increases effectiveness of medical monitoring and treatment.

According to the present invention, a repositionable medical device for application to the skin of a user is provided comprising a carrier layer and a functional unit. The carrier layer includes an application surface configured to be directed towards the skin when applied to the user to ensure correct application of the device. Preferably, the material of the carrier layer should be a soft-feeling material such as a nonwoven, thermoplastic polyurethane or foam, to minimize stress to the skin surface.

The functional unit is configured to operate in relation to the user. In the context of the present invention as disclosed herein, the term "operate in relation to the user" refers to the ability of the functional unit to interact with, monitor, or influence physiological parameters or signals of the user. This includes, but is not limited to, sensing biological signals such as electrical activity, temperature, hydration levels, or biochemical markers, delivering therapeutic agents or electrical stimulation, and facilitating data transmission for medical monitoring purposes. The functional unit may therefore include a sensor, an electrode element (i.e., electrically conductive element; sometimes simply referred to as "electrode"), a transdermal drug delivery mechanism, or a communication module, depending on the specific application of the medical device.

The functional unit is either embedded within the carrier layer or positioned on the application surface. For example, if the functional unit is the electrode element, the carrier layer itself may comprise a conductive element, such as dry electrode material, or the conductive element may be attached to the application surface, which may allow the conductive element to be directly or indirectly in contact with the user's skin when the device is applied. The dry electrode material for example comprises conductive plastic, in particular, Ag/AgCl plastic.

The repositionable medical device further comprises a release layer having a first release surface and a second release surface. The second release surface is configured to adhere to the application surface of the carrier layer; however it may also be configured to be detachable from said application surface. This adhesion can be achieved through various means, such as a permanently adherent adhesive, a pressure sensitive adhesive, Vanderwal's forces, or a material that passively adheres to the carrier layer without the need for additional bonding agents.

The first release surface of the release layer may be oriented in the same direction as the application surface and can be configured in two different ways, depending on the configuration of the device. In a first configuration (i), the first release surface is configured to adhere directly to the user's skin when applied. This means that when the device is placed on the skin, the first release surface secures the device in place. When the release layer is detached, it exposes an adhesive portion of the application surface, which then allows the device to be reapplied to the skin. Preferably, the material of the release layer comprises material that does not contaminate the skin adhesive of the carrier layer, such as thermoplastic polyurethane, or other smooth, non-porous films that minimize particle transfer and do not absorb or degrade the adhesive properties over time. This configuration can be implemented with a single release layer and is particularly beneficial for medical devices that require a very thin build up.

In a second configuration (ii), the first release surface does not adhere directly to the skin but instead adheres to an additional release layer. In this case, the at least one additional release layer serves as an intermediary between the primary release layer and the user's skin. In general, additional release layers have the same build up as the primary release layer and are detachable from the first release surface of the primary or any additional release surface. Detachment of the additional release layer exposes the first release surface of the primary release layer, allowing the medical device to be reapplied to the user's skin. This configuration provides an additional layer and enables multiple applications of the same device by peeling away consecutive release layers. Thus, in this configuration the device comprises at least two release layers.

The repositionable medical device may take various forms, including but not limited to a dry electrode, a wearable device, a vital signs measurement device, a drug delivery patch, a biosensing patch, an electrophysiological sensing patch, or a wearable therapeutic patch. Wearable therapeutic patches may include electrical stimulation devices, pain relief patches, or neuromodulation systems, which may require precise positioning over target nerves or muscles. This broad scope of application ensures the versatility of the invention and its applicability to a wide range of fields in medical technology.

Preferably, the functional unit may be configured to perform at least one of physiological monitoring of the user, measuring one or more physiological signals of the user, drug delivery to the user, and bioelectrical interaction with the user. This means that the repositionable medical device can perform a variety of critical healthcare functions, depending on its specific design and application. In the context of physiological monitoring, the functional unit may include the electrode element or a sensor that tracks vital signs such as heart rate, respiration, temperature, hydration levels, glucose levels, or electrical activity of the body. This enables real-time health tracking, making the device valuable for both clinical and home patient monitoring.

In applications related to drug delivery, the functional unit may facilitate controlled administration of medication through the skin, such as in transdermal patches used for pain management, hormone therapy, or chronic disease treatment. By ensuring precise and consistent drug release through proper positioning, the invention enhances treatment efficacy while reducing side effects associated with fluctuating drug levels. In addition, in bioelectric interaction, the functional unit may include an electrically active element that delivers electrical stimulation for therapeutic purposes. Such devices rely on precise electrode placement to function effectively, and the ability to reposition the device without losing adhesion significantly improves usability and patient comfort.

In a preferred embodiment, the functional unit may be exposed directly to the user's skin through a recess or an opening in the release layer, providing better contact with the skin. This configuration allows the functional unit to maintain direct contact with the skin of the user. Such direct exposure is particularly advantageous for applications that require precise physiological monitoring, drug delivery, or bioelectrical interaction.

In an embodiment, the release layer may comprise an additional functional unit that is either positioned on the first release surface or embedded within the release layer itself. This design allows the release layer to contribute actively to the device's overall function rather than serving solely as an adhesive interface. For example, in the case of dry electrodes, the release layer may include conductive elements that facilitate electrical signal transmission between the user's skin and the primary functional unit. This ensures that even when the device is repositioned, electrical conductivity is maintained, improving the reliability of electrophysiological monitoring or bioelectrical stimulation.

In another embodiment, the application surface of the carrier layer may itself be configured to adhere to the skin, eliminating the need for a separate adhesive layer in certain configurations. By integrating the adhesive directly into the application surface, the need for the additional release layer can be eliminated, simplifying the device's design and potentially lowering manufacturing costs.

In another embodiment, the repositionable medical device may further comprise one or more additional release layers arranged on top of the primary release layer, each featuring a first release surface configured to adhere to the skin and a second release surface configured to adhere to the adjacent release layer. It is understood that the release layer positioned closest to the application surface will adhere to this application surface, ensuring a secure connection between the carrier layer and the stack of release layers. This layered configuration allows for multiple repositioning cycles, as the user can remove one release layer at a time to expose a fresh adhesive surface for reapplication.

The carrier layer and/or release layer may comprise a breathable material configured to facilitate air exchange and/or wick moisture away from the user's skin. This allows air to circulate between the skin and the device, preventing the build-up of unwanted moisture. Breathable materials such as nonwoven fabrics, perforated foams or microporous films may be used to help regulate skin temperature and reduce the accumulation of sweat, which could otherwise affect adhesion. **In** addition, moisture-wicking properties actively draw sweat away from the skin, keeping the contact area dry and stable. This improves adhesion consistency, minimizes discomfort and reduces the risk of irritation, making the device more suitable for long-term wear.

In an embodiment, the release layer may further comprise a release lip at its outer edge, which extends beyond the edge of the carrier layer and is specifically configured to facilitate the removal of the release layer from the application surface. It may be located at different positions of each release layer to increase user accessibility. This release lip provides a designated gripping area, allowing the user or healthcare provider to easily lift and peel away the release layer without damaging the adhesive or disturbing the functional unit. By extending beyond the carrier layer, the release lip remains accessible even when the device is fully applied to the skin, ensuring smooth and controlled removal without requiring excessive force.

In yet another embodiment, the repositionable medical device may further comprise a cover layer adhered to the first release surface of the release layer. This cover layer is configured to be detachable, thereby exposing the first release surface when removed. This cover layer serves as a protective barrier, shielding the adhesive surface from contamination, dust, or premature adhesion before the device is applied to the user's skin. By keeping the adhesive layer clean and intact, the cover layer ensures optimal adhesion performance when the device is first applied.

In another embodiment the repositionable medical device may be a dry electrode, and the functional unit comprises a conductive layer that enables electrical signal transmission between the user's skin and external medical equipment. To ensure efficient connectivity, the device further includes an adapter with a contact layer that is in contact with the conductive layer, and it may comprise a connection element configured to connect to a cable. This configuration allows the electrode to be easily integrated into systems such as ECG, EMG, EEG, or neuromodulation devices, ensuring stable signal acquisition or electrical stimulation. By providing a secure and adaptable connection, this embodiment enhances the overall usability of the device in medical environments.

In a preferred embodiment, the adhesive strength between the first release surface and the user's skin may be lower than the adhesive strength between the adhesive portion of the application surface and the user's skin. This means that while the release layer ensures secure initial attachment, it can be easily removed or replaced without causing discomfort or leaving excessive residue. In contrast, the application surface of the carrier layer is configured to provide a stronger and more durable adhesion, ensuring that the device remains in place during prolonged wear or repositioning. This controlled difference in adhesive strength allows for multiple applications of the device while maintaining stability, as the carrier layer remains adhered even if the release layer is detached and replaced.

### BRIEF DESCRIPTION OF THE DRAWINGS

These and other aspects of the invention will be apparent from and elucidated with reference to the embodiments described hereinafter. In the following drawings:
Fig. 1 shows a schematic illustration of a first embodiment of a device according to the present invention;
Fig. 2 shows a schematic illustration of the device of Fig. 1 in an assembled state;
Fig. 3 shows a schematic illustration of a user with an applied device according to the present invention;
Fig. 4 shows a schematic illustration of a second embodiment of a device according to the present invention;
Fig. 5 shows a schematic illustration of a third embodiment of a device according to the present invention;
Fig. 6 shows a schematic illustration of a fourth embodiment of a device according to the present invention;
Fig. 7 shows a schematic illustration of a fifth embodiment of a device according to the present invention;

### DETAILED DESCRIPTION OF EMBODIMENTS

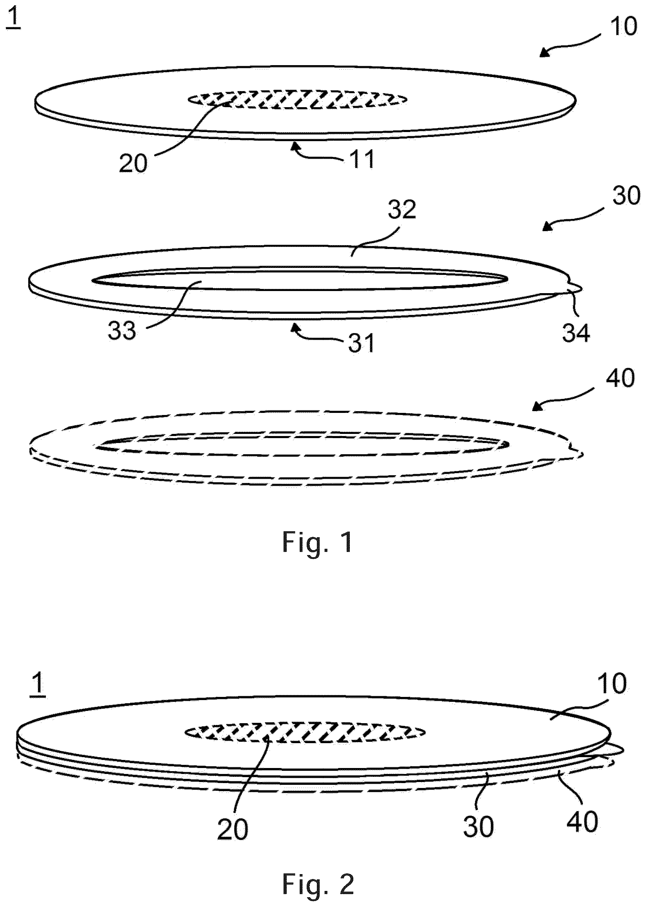

Fig. 1 shows a schematic illustration of a first embodiment of a repositionable medical device 1 according to the present invention in a disassembled state to show the different layers in detail. The device 1 comprises a carrier layer 10 having an application surface 11 that is configured to be directed towards the skin when applied to a user 2 (not shown in this figure). In the center of the application surface a functional unit 20 (e.g. an electrode element) is positioned, which is configured to interact with, monitor or influence physiological parameters of the user 2. Since the surface of the application surface 11 is not shown, the position of the functional unit 20 is indicated by a dashed outline.

The device 1 further comprises a release layer 30 having a first release surface 31, a second release surface 32, a recess (or opening) 33, and a release lip 34, wherein the second release surface 32 is configured to adhere to the application surface 11 yet be detachable therefrom. The recess 33 allows the functional unit 20 to be in contact with the user's 2 skin, and the release lip 34 facilitates gripping of the release layer 30. However, both the recess 33 and the release lip 34 are optional features of the device yet provide useful functions.

The first release surface 31 of the release layer 30 may be configured either to adhere directly to the skin of a user 2, or to adhere to an additional release layer 40 and adhere to the skin of the user 2 upon detachment of the additional release layer 40. The application surface 11, or at least a portion of the application surface 11, may also comprise adhesive components so that when the release layer 30 is removed, the carrier layer 10 can be reapplied to the skin, thereby enabling the repositionability of the device 1.

In Fig. 2, the layers of the repositionable medical device 1 are shown in their assembled state, meaning that they are depicted as being adhered together rather than separated as in Fig. 1. This representation illustrates how the various layers interact when the device 1 is in use to ensure proper functionality and stability on the skin. In particular, the carrier layer 10, the release layer 30 and, if present, the additional release layer 40 are shown in direct contact as they would be in an actual application.

While the individual components were separated in Fig. 1 for clarity, all embodiments of the invention ultimately appear in a similar assembled manner when applied to the user 2. The separation of layers in other figures is primarily for illustrative purposes, to provide a clearer understanding of the structure and interaction of the components of the device 1. In practice, however, the layers adhere together until the release layer 30 (or the additional release layer 40) is intentionally removed to expose another adhesive surface (31 or 11) and allow repositioning on the skin of the user 2. All layers may have a thin structure of about 50 µm in thickness to improve user comfort.

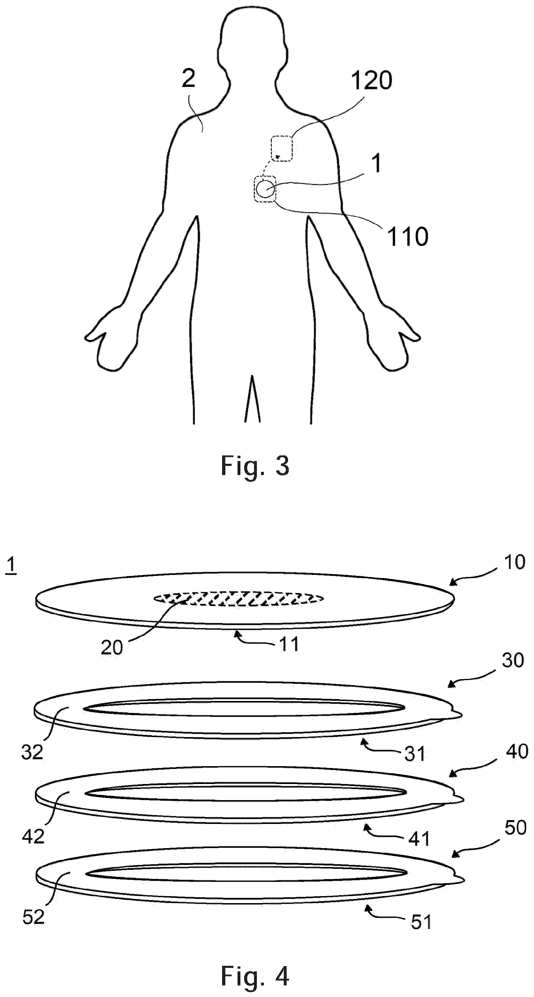

Fig. 3 shows a schematic illustration of a user 2 with an applied device 1, according to the present invention, being placed in a first position 110. If, after application in the first position 110, it is necessary to place the device in a second position 120, the user 2 or medical staff may remove the device, tear off the additional release layer 40 or release layer 30 (not shown in this figure), and reposition the device in the second position 120. As the device is removed from the first position 110, the additional release layer 40 or release layer 30 can be peeled away, exposing a fresh adhesive surface that allows for secure repositioning in the second position 120. This enables repositioning without compromising the adhesive properties of the device. Repositioning may be necessary if the initial placement does not provide optimal interaction capabilities or skin contact, thereby compromising sensor accuracy, drug absorption, or therapeutic efficacy. Additionally, it may be required if the user 2 experiences discomfort or skin irritation, or if medical personnel determine that a different location is more appropriate due to changes in the patient's condition or treatment requirements.

Fig. 4 shows a schematic illustration of a second embodiment of a device 1 according to the present invention. This embodiment includes a carrier layer 10 with a functional unit 20, a first release layer 30 and a second release layer 40, similar to the first embodiment. However, this embodiment additionally includes a third release layer 50. In total, the embodiment comprises three release layers (30, 40, 50), each having a first release surface (31, 41, 51) and a second release surface (32, 42, 52).

Starting from the outermost layer, the first release surface 51 of the third release layer 50 is configured to adhere to the skin of the user 2, while its second release surface 52 adheres to the first release surface 41 of the second release layer 40. When the third release layer 50 is removed, it exposes the first release surface 41 of the second release layer 40, which can then adhere to the skin. This process may be repeated in sequence, with the second release layer 40 being removed to expose the first release surface 31 of the first release layer 30, which may then adhere to the skin. Finally, removal of the first release layer 30 exposes the adhesive portion of the carrier layer 10, allowing further secure attachment of the device 1.

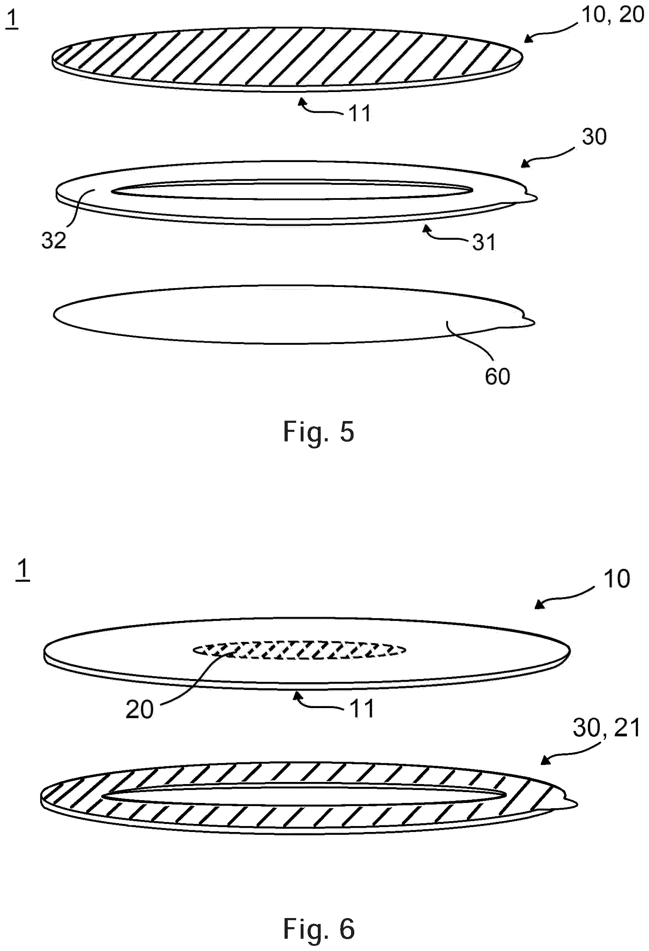

Fig. 5 shows a schematic illustration of a third embodiment of a device 1 according to the present invention, wherein the whole carrier layer 10 comprises the functional unit 20 or, in other words, wherein the carrier layer 10 is the functional unit 20. In this configuration, the carrier layer 10 itself performs the primary function of the device 1, eliminating the need for a separately embedded or attached functional unit 20. This can be achieved, for example, by manufacturing the carrier layer 10 from a conductive, sensing, or drug-infused material, depending on the intended application. For instance, in the case of a dry electrode, the carrier layer 10 may be made from a conductive polymer or coated with a conductive layer, allowing direct electrical interaction with the skin. Using this integrated approach reduces the number of components, simplifies manufacturing, and increases the flexibility and comfort of the device 1 while maintaining its intended functionality.

This third embodiment further comprises a cover layer 60 adhered to, but removable from, the first release surface 31 of the release layer 30, wherein removal of the cover layer 60 exposes the first release surface 31 of the release layer 30. The cover layer 60 serves as a protective barrier to prevent contamination or premature adhesion of the first release surface 31 prior to application. It may be made of a flexible film, paper, or other removable material that is easily detachable when the device is ready for use. The adhesive strength between the cover layer 60 and the first release surface 31 is typically weaker than the adhesive strength between the first release surface 31 and the skin, ensuring smooth removal without damaging or impairing the adhesive properties of the release layer 30.

Fig. 6 shows a schematic illustration of a fourth embodiment of a device according to the present invention, wherein the whole release layer 30 comprises an additional functional unit 21 or, in other words, wherein the release layer 30 itself serves as the additional functional unit 21. In this configuration, the release layer 30 not only provides an adhesive interface but also actively contributes to the functionality of the device. This can be achieved by integrating conductive, sensing, or therapeutic materials directly into the release layer 30. For example, in a dry electrode application, the release layer 30 may be a conductive film or coated with conductive elements to allow it to contribute to electrical signal transmission.

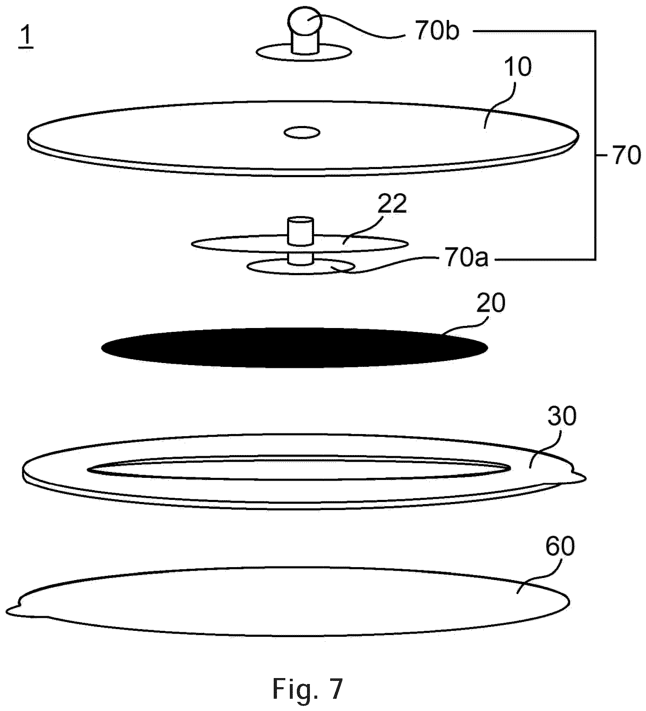

Fig. 7 shows a schematic illustration of a fifth embodiment of a device 1 according to the present invention, wherein the device 1 is a dry electrode. The dry electrode comprises the carrier layer 10 comprising skin adhesive, the functional unit 20 comprising a conductive layer (e.g. an electrode element), the release layer 30, and the cover layer 60, similar to previous embodiments. The dry electrode further comprises an adapter 70 that facilitates external connection. The dry electrode may further comprise a layer of printed electronics 22. The printed electronics 22 serves as an integrated circuit layer that provides efficient signal routing and processing, enhancing the overall performance of the electrode. In particular, the diameter of the printed electronics 22 is slightly smaller than the diameter of the dry electrode material of the functional unit 20, ensuring proper alignment and integration within the structure. The outer edge of the functional unit 20 can be positioned directly onto the skin adhesive of the carrier layer 10, effectively securing it in place. Consequently, the inner diameter (opening) of the release layer 30 matches the diameter of the functional unit 20 to ensure a proper fit and alignment when applied.

The adapter 70 comprises a contact layer 70a that is positioned in contact with the functional unit 20, providing stable electrical conduction from the electrode to the external system. A connection element 70b, e.g. a stud, or a tab, may be integrated into the adapter to allow a cable to be attached for connection to medical monitoring or stimulation devices. Instead of the adapter 70 having a connection element 70b, the contact layer 70a can be directly connected to a cable.

Dry electrode technology already offers significant advantages over hydrogel-based electrodes, including extended wear (10-15 days instead of 3-7), reduced skin irritation, and reduced environmental impact. However, repositionability has been a highly requested feature, especially given the higher cost of dry electrodes compared to wet electrodes. Since repositionability is primarily a function of the skin adhesive rather than the electrode itself, the invention may employ different adhesive technologies, including silicones, acrylates, and rubbers, to optimize adhesion dynamics. While silicones provide instant, stable adhesion, acrylates build adhesion strength over time, and rubbers provide strong initial adhesion that gradually degrades. By incorporating a controlled release mechanism, the invention ensures that the adhesive remains effective despite exposure to skin contaminants, allowing nurses and caregivers to reposition the device for better placement, improved patient comfort and extended usability. This innovation significantly improves the practicality of wearable medical devices, making them more reliable, cost-effective, and user-friendly for both patients and healthcare providers. Furthermore, the technology of the invention can be used for patches such as ePatch, MCOT, iRythm or VitalConnect of the applicant.

There are a total of three different mechanical-electrical connections, in which the device 1, in particular the adapter 70, may be configured. The most common is the snap connector, which is a small stub on the outside of the electrode allowing a snap or grab connector to interface (as shown in Fig. 7). The advantage is that they are both inexpensive and well accepted in the medical field with a standardized interface. Another option is a pre-wired electrode, where a cable runs from the electrode to a standardized medical connector. The advantages of these electrodes are a more secure connection (compared to the snap-on electrode) and the elimination of pressure points (when using a snap-on connector). They are mainly used for long-term monitoring and in fragile patients (e.g. neonates). The third option is the tab electrode, which is the least expensive option and requires a clamp-type connector. Another advantage is that it eliminates pressure points. They are mainly used for very short electrophysiological studies.

**In** summary, the present invention allows for repositioning of medical adhesive devices without compromising adhesion, functionality, or user comfort. By incorporating a multi-layered release system, the invention ensures that the device can be securely applied, removed, and reapplied while maintaining reliable skin contact and adhesive performance.

The invention can be used in various medical devices, including dry electrodes, biosensors, measuring units, drug delivery patches, and wearable therapeutic devices, making it highly versatile across multiple healthcare applications. The combination of removable adhesive layers, integrated functional units, and optional connectivity features enhances both ease of use and long-term performance.

While the invention has been illustrated and described in detail in the drawings and foregoing description, such illustrations and descriptions are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A repositionable medical device for application to a user's skin comprising:
- a carrier layer having an application surface configured to be directed towards the skin when applied to the user,
- a functional unit embedded within the carrier layer or positioned on the application surface, and
- a release layer having a first release surface and a second release surface;
wherein the second release surface is configured to adhere to the application surface of the carrier layer, and
wherein the first release surface is configured to adhere to:
i) the skin when applied to the user, wherein detachment of the release layer exposes an adhesive portion of the application surface, or
ii) an additional release layer, wherein detachment of the additional release layer exposes the first release surface allowing a renewed application of the medical device to the user's skin.

2. The repositionable medical device according to claim 1,
wherein the repositionable medical device is a dry electrode, a wearable, a vital signs measurement device, a drug delivery patch, a biosensing patch, an electrophysiological sensing patch or a wearable therapeutic patch.

3. The repositionable medical device according to any preceding claim,
wherein the functional unit is configured to perform at least one of physiological monitoring of the user, measuring one or more physiological signal of the user, drug delivery to the user, and bioelectrical interaction with the user.

4. The repositionable medical device according to any preceding claim,
wherein the release layer comprises a recess or an opening configured to expose the functional unit to the skin when applied to the user.

5. The repositionable medical device according to any preceding claim,
wherein the release layer comprises an additional functional unit positioned on the first release surface or embedded within the release layer.

6. The repositionable medical device according to any preceding claim,
wherein the application surface of the carrier layer is configured to adhere to the skin after detachment of the release layer.

7. The repositionable medical device according to any preceding claim,
further comprising one or more additional release layers arranged on top of the release layer, each additional release layer having a first release surface configured to adhere to the skin when applied to the user and a second release surface configured to adhere to an adjacent release layer.

8. The repositionable medical device according to any preceding claim,
wherein the carrier layer comprises breathable material configured to facilitate air exchange and/or wick moisture away from the skin of the user.

9. The repositionable medical device according to any preceding claim,
wherein the release layer comprises breathable material configured to facilitate air exchange and/or wick moisture away from the skin of the user.

10. The repositionable medical device according to any preceding claim,
wherein the release layer further comprises a release lip at its outer edge configured to facilitate removal of the release layer from the application surface, and
wherein the release lip extends beyond an edge of the carrier layer.

11. The repositionable medical device according to any preceding claim,
further comprising a cover layer adhered to the first release surface of the release layer,
wherein the cover layer is detachable from the first release surface of the release layer, and
wherein detachment of the cover layer exposes the first release surface of the release layer.

12. The repositionable medical device according to any preceding claim,
wherein the repositionable medical device is a dry electrode and the functional unit comprises a conductive layer, and
wherein the repositionable medical device further comprises an adapter having a contact layer in contact with the conductive layer, wherein the adapter is configured to electrically connect to a cable.

13. The repositionable medical device according to any preceding claims,
wherein the adhesive strength between the first release surface and the user's skin is lower than the adhesive strength between the adhesive portion of the application surface and the user's skin.
